# FASCICULE DE BREVET EUROPEEN

(11) **EP 1 370 192 B1**
(45) Date de publication et mention de la délivrance du brevet: **29.06.2005**
(21) Numéro de dépôt: 02716887.1
(22) Date de dépôt: 08.03.2002
(51) Int. Cl.: A61F 2/00

(54) **DISPOSITIF FAVORISANT ET RENFORCANT L'ERECTION DE LA VERGE**
VORRICHTUNG ZUM FÖRDERN UND VERSTÄRKEN DER PENISEREKTION
DEVICE FACILITATING AND REINFORCING AN ERECTION OF THE PENIS

(30) Priorité: 16.03.2001 FR 0103649
(43) Date de publication de la demande: 17.12.2003
(73) Titulaire: Benestar Aura SL, Ransol (Canillo) (AD)
(72) Inventeur: Egretier, Jean-Michel, 11110 Coursan (FR)
(74) Mandataire: Gosse, Michel
(86) Numéro de dépôt international: PCT/FR2002/000846
(87) Numéro de publication internationale: WO 2002/074187

(56) Documents cités:
- DE-A- 3 606 126
- FR-A- 2 736 539
- US-A- 4 995 381
- US-A- 5 327 910
- US-A- 5 997 469

## Description

L'invention concerne un dispositif destiné à favoriser et à renforcer l'érection de la verge par un effet d'amorçage préalable à l'érection proprement dite et de pompage lors de l'acte sexuel.

Les mécanismes de l'érection ont pour point de départ un centre nerveux qui est situé dans la moelle épinière (colonne vertébrale). Le cerveau a un effet modulateur sur les centres nerveux de l'érection. Les fibres qui innervent la verge descendent dans le petit bassin et longent les bords des vésicules séminales et de la prostate (les nerfs caverneux), puis l'urètre membraneux. Les branches terminales des nerfs caverneux innervent les artères des corps caverneux et les fibres musculaires lisses, contrôlant ainsi la tumescence (remplissage) et la détumescence (retour à l'état normal) de la verge.
La verge contient 3 structures qui permettent l'érection car elles contiennent du tissu érectile : 2 corps caverneux qui sont des cylindres parallèles situés au centre de la verge, et le corps spongieux qui contient l'urètre et qui constitue l'essentiel du gland.
Le tissu érectile est fait de vaisseaux sanguins organisés de façon assez lâche et entourés de fibres musculaires lisses. Au repos, la verge est molle et l'arrivée de sang dans les corps caverneux est réduite au minimum car les vaisseaux sanguins sont contractés. Lors d'une érection, l'excitation sexuelle entraîne un signal nerveux qui fait relaxer le muscle lisse présent autour des vaisseaux sanguins du tissu érectile. Le sang peut alors remplir et dilater ces vaisseaux qui sont mis sous pression à l'intérieur de la paroi rigide des corps caverneux (les deux corps caverneux sont entourés par l'albuginée, cloison dure et non distensible). Les veines empêchent le sang de s'échapper, ce qui entraîne une mise sous tension et l'état d'érection de la verge. La rigidité de l'érection dépend alors de l'apport artériel et de l'efficacité de l'occlusion veineuse dans les corps caverneux. La détumescence survient par rétablissement du drainage veineux des corps caverneux (recontraction par stimulation nerveuse).
Les muscles bulbo-caverneux et ischio-caverneux assurent, en se contractant, une augmentation de la pression intra-cavemeuse et donc de l'érection.
Trois phénomènes se succèdent donc :
- la relaxation du tissu érectile;
- la vasodilatation et l'augmentation du débit artériel;
- le blocage du retour veineux sous l'albuginée.

Le dispositif selon l'invention est le résultat de l'analyse anatomique et physiologique des mécanismes de l'érection.
Des dispositifs selon le préambule de la revendication 1 sont décrits dans les documents US 4 995 381 et DE 36 06 126.
Contrairement aux dispositifs connus des types anneaux peu ou pas élastiques qui bloquent totalement le retour sanguin après érection et qui sont placés à la base de la verge, le dispositif selon l'invention agit comme une pompe à clapet qui, à chaque pression (avant l'érection proprement dite et lors du rapprochement des partenaires en présence), permet le passage du sang vers la verge renforçant ainsi la rigidité de celle-ci et, à chaque dépression (écartement), permet le freinage du retour sanguin.
Cet effet a été obtenu grâce à la forme donnée audit dispositif, à sa conception et à son fonctionnement lors des mouvements de va-et-vient qu'il subit.
Le dispositif selon l'invention, destiné à favoriser et à renforcer l'érection de la verge, réalisé en matériau élastique de forme générale annulaire, adapté pour être placé dans la zone de la base de la verge et pour exercer une pression périphérique bien déterminée sur celle-ci ; se caractérise :
- en ce que son corps de révolution, qui présente une face frontale servant de surface d'appui avec la zone périphérique vaginale externe de la partenaire et une face interne servant de surface d'appui avec la verge, comporte, en zone inférieure, une partie prolongatrice, fraction cylindrique, dont la face interne vient dans le prolongement de la face interne inférieure dudit corps de révolution ;
- en ce que le matériau constitutif du volume occupé par ledit corps de révolution et sa partie prolongatrice, est adapté pour transmettre toute déformation négative subie, sous l'effet d'une compression, par sa face frontale en une déformation positive de la paroi interne inférieure dudit corps et de sa partie prolongatrice, qui comprime ainsi la partie inférieure de la verge ;
- et en ce que le corps de révolution et sa partie prolongatrice effectuent, à chaque pression sur sa face frontale inférieure, un mouvement de recul et de basculement.
Ledit dispositif se place dans la zone de la base de la verge en lui laissant une certaine liberté de mouvement pour que les effets de pompage et de frein se fassent en toute liberté.
C'est ce mouvement associé à la déformation de la paroi inférieure du corps de révolution qui permet de comprimer la partie inférieure de la verge et de décomprimer sa partie supérieure et ce de manière alternative.
La partie inférieure du corps de révolution comporte une prolongation, de forme générale fraction cylindrique, apte, par sa face interne, à amplifier la déformation positive de la paroi inférieure dudit dispositif au contact de la partie inférieure de la verge.

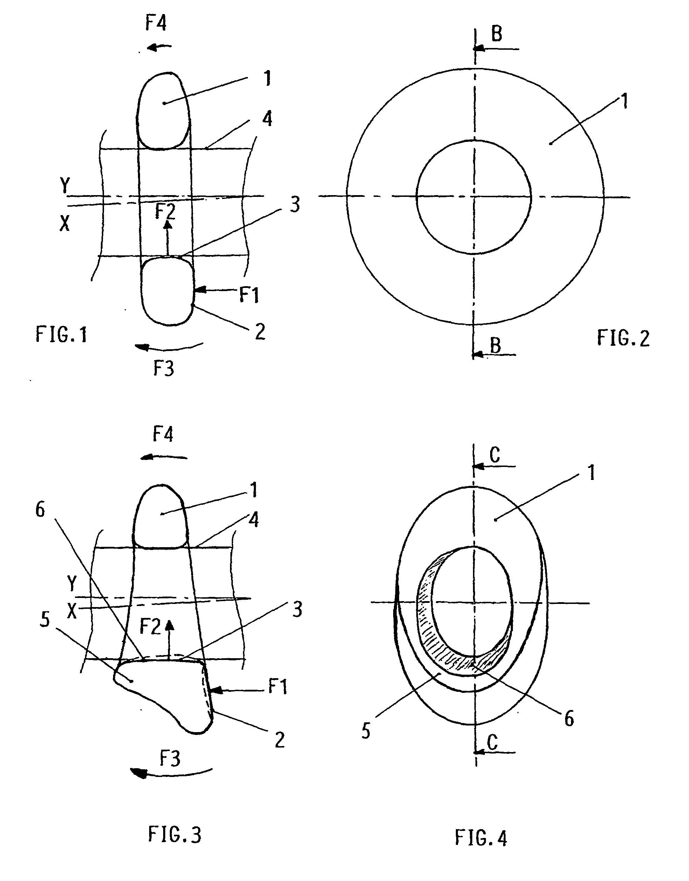

Les caractéristiques et les avantages de l'invention vont apparaître plus clairement à la lecture de la description détaillée qui suit d'au moins un mode de réalisation préféré de celle-ci donné à titre d'exemple non-limitatif et représenté aux dessins annexés.
Sur ces dessins :
- la figure 1 est une vue en coupe longitudinale, selon l'axe (BB), d'un dispositif qui n'est pas selon l'invention de la revendication 1;
- la figure 2 est une vue frontale dudit dispositif de base;
- la figure 3 est une vue en coupe longitudinale, selon l'axe (CC), du dispositif comportant une prolongation selon l'invention;
- la figure 4 est une vue en perspective dudit dispositif.
Le dispositif représenté aux figures 3 et 4 est destiné à favoriser et à renforcer l'érection de la verge, est réalisé en matériau élastique de forme générale annulaire et est adapté pour être placé dans la zone de la base de la verge et pour exercer une pression périphérique bien déterminée sur celle-ci.
Son corps de révolution (1), qui présente une face frontale (2) servant de surface d'appui avec la zone périphérique vaginale externe de la partenaire et une face interne (3) servant de surface d'appui avec la verge (4), comporte, en zone inférieure, une partie prolongatrice (5), fraction cylindrique, dont la face interne (6) vient dans le prolongement de la face interne inférieure (3) du corps de révolution (1).
Le matériau constitutif du volume occupé par ledit corps de révolution (1) et sa partie prolongatrice (5), est adapté pour transmettre toute déformation négative subie, sous l'effet d'une compression (F1), par la face frontale (2) en une déformation positive (F2) de la paroi interne inférieure (3,6) dudit corps (1) et de sa partie prolongatrice (5), qui comprime ainsi la partie inférieure de la verge (4).
La face interne (6) de la partie prolongatrice (5) amplifie la déformation positive de la paroi intérieure du dispositif selon l'invention.
Le corps de révolution (1) et sa partie prolongatrice (5) effectuent, à chaque pression sur la face frontale (2) inférieure, un mouvement de recul et de basculement selon les flèches (F3) et (F4).
Pour une meilleure utilisation dudit dispositif, l'axe (X) de l'ouverture de la surface interne (3) de celui-ci est incliné vers l'arrière par rapport à l'axe (Y) tangentiel de la verge dans la zone de ladite ouverture.
La mise en place et l'amorçage se font de la manière suivante :
- le corps (1) est placé à la base de la verge au repos à une distance de 1 à 2 cm de sa terminaison visible;
- le déplacement de celui-ci vers l'arrière entraîne le sang qui gonfle la partie amont qui induit un signal nerveux qui fait relayer le muscle lisse avec pour conséquence un début de remplissage des vaisseaux sanguins de la partie aval des corps caverneux;
- le basculement alternatif du corps (1) selon les flèches (F3,F4) permet d'amorcer le phénomène d'érection;
- le mouvement dudit corps pendant l'acte sexuel agit comme une pompe à clapet.
L'étranglement dû au diamètre intérieur dudit corps n'est pas agressif et ne doit pas agir comme un garot.
Le dispositif peut être réalisé :
- en un matériau souple obtenu par moulage ou par toute autre technique;
- au moyen d'une poche remplie d'air, de liquide ou d'un matériau souple, peu ou pas compressible du type gel ou autre.
La section du corps (1) peut être ovoïde.

Bien entendu, l'invention n'est pas limitée aux modes de réalisation décrits et représentés pour lesquels on pourra prévoir d'autres variantes, limitées par les revendication suivantes, en particulier dans :
- les formes et les dimensions du corps de révolution et de sa protubérance;
- la nature des matériaux utilisés;
- les techniques de réalisation du dispositif.

## Revendications

1. Dispositif destiné à favoriser et à renforcer l'érection de la verge, réalisé en matériau élastique de forme générale annulaire, adapté pour être placé dans la zone de la base de la verge et pour exercer une pression périphérique bien déterminée sur celle-ci ; **caractérisé :**
- **en ce que** son corps de révolution (1), qui présente une face frontale (2) servant de surface d'appui avec la zone périphérique vaginale externe de la partenaire et une face interne (3) servant de surface d'appui avec la verge (4), comporte, en zone inférieure, une partie prolongatrice (5), de forme générale fraction cylindrique, dont la face interne (6) vient dans le prolongement de la face interne inférieure (3) du corps de révolution (1) ;
- **en ce que** le matériau constitutif du volume occupé par ledit corps de révolution (1) et sa partie prolongatrice (5), est adapté pour transmettre toute déformation négative subie, sous l'effet d'une compression (F1), par la face frontale (2) en une déformation positive (F2) de la paroi interne inférieure (3,6) dudit corps (1) et de sa partie prolongatrice (5), qui comprime ainsi la partie inférieure de la verge (4) ;
- et **en ce que** le corps de révolution (1) et sa partie prolongatrice (5) effectuent, à chaque pression sur la face frontale (2) inférieure, un mouvement de recul et de basculement vers la base de la verge.

2. Dispositif, selon la revendication 1, **caractérisé en ce que** l'axe (X) de l'ouverture de sa surface interne (3) est incliné vers l'arrière par rapport à l'axe (Y) tangentiel de la verge dans la zone de ladite ouverture.

3. Dispositif, selon la revendication 1 ou la revendication 2, **caractérisé en ce qu'**il est réalisé en un matériau souple obtenu par moulage.

4. Dispositif, selon la revendication 1 ou la revendication 2, **caractérisé en ce qu'**il est réalisé au moyen d'une poche remplie d'air, de liquide ou d'un matériau souple.

## Patentansprüche

1. Vorrichtung zur Förderung und Unterstützung der Erektion des Glieds, ausgeführt aus einem Material mit einer generell ringförmigen Form, das an der Basis des Glieds angebracht werden kann und einen bestimmten Umfangsdruck auf dieses ausüben soll, **dadurch gekennzeichnet:**
- **dass** ihr Drehkörper (1) mit einer Stirnfläche (2), die als Anlagefläche zum äußeren vaginalen Bereich der Partnerin dient, und mit einer inneren Fläche (3), die als Anlagefläche für das Glied (4) dient, im unteren Bereich einen generell zylindrischen Verlängerungsabschnitt (5) aufweist, dessen Innenfläche (6) sich in die Verlängerung der unteren Innenfläche (3) des Drehkörpers (1) erstreckt;
- **dass** das Material des vom Drehkörper (1) geformten Volumens und dessen Verlängerungsabschnitt (5) für die Umwandlung jeglicher auftretender negativen Verformungen in Form einer Kompression (F1) auf der Stirnseite (2) in eine positive Verformung (F2) der unteren Innenwand (3,6) dieses Körpers (1) und des Verlängerungsabschnitts (5) geeignet ist, wodurch der unteren Bereich des Glieds (4) zusammengedrückt wird;
- und **dass** der Drehkörper (1) und sein Verlängerungsabschnitt (5) bei jedem Druck auf seine untere Stirnseite (2) eine Rückwärts- und Schwenkbewegung zur Basis des Glieds hin ausführt.

2. Vorrichtung gemäß dem Patentanspruch 1 **dadurch gekennzeichnet, dass** die Achse (X) der Öffnung ihrer Innenfläche (3) in Bezug auf die Tangentialachse (Y) des Glieds im Bereich dieser Öffnung nach hinten geneigt ist.

3. Vorrichtung gemäß dem Patentanspruch 1 bzw. 2 **dadurch gekennzeichnet, dass** sie aus einem Spritzgussmaterial hergestellt ist.

4. Vorrichtung gemäß dem Patentanspruch 1 bzw. 2 **dadurch gekennzeichnet, dass** sie mittels eines mit Luft, einer Flüssigkeit oder einem Spritzgussmaterial gefüllten Beutels gebildet wird.

## Claims

1. Device designed to facilitate and strengthen the penis erection, made of generally ring-shaped material, adapted to be placed in the area at the basis of the penis and to exert a well-determined peripheral pressure on it, **characterized in that**:
- its revolution body (1) showing a front face (2) which serves as supporting surface with the peripheral vaginal external area of the partner and an internal face (3) serving as supporting surface with the penis (4) includes, at the lower area, an extending part (5), in general of a cylindrical fraction shape, the internal face (6) of which is extending the lower internal face (3) of the revolution body (1);
- the material constituting the volume occupied by such revolution body (1) ant its extending part (5) is adapted to transmit any negative deformation sustained under the effect of a compression (F1) by the front face (2) to a positive deformation (F2) of the lower internal wall (3, 6) of such body (1) and of its extending part (5) which compresses thus the lower part of the penis (4);
- the revolution body (1) and its extending part (5) carries out at each pressure on the lower front face (2) a back and tilt motion towards the base of the penis.

2. Device, according to claim 1, **characterized in that** the axis (X) of the opening of its internal surface (3) is tilt backwards with respect to the axis (Y) tangential to the penis in the area of such opening.

3. Device, according to the claim 1 or the claim 2, **characterized in that** it is made of a flexible material obtained by moulding it.

4. Device, according to claim 1 or to claim 2 **characterized in that** it is made of a pocket full of air, liquid or a flexible material.
